# EUROPEAN PATENT SPECIFICATION

(11) **EP 3 562 394 B1**
(45) Date of publication and mention of the grant of the patent: **26.07.2023**
(21) Application number: 16829406.4
(22) Date of filing: 28.12.2016
(51) Int. Cl.: A61B 5/08, A61B 5/11, A61B 5/113, A61B 5/18

(54) **RESPIRATORY SIGNAL EXTRACTION**
ATEMSIGNALEXTRAKTION
EXTRACTION DE SIGNAL RESPIRATOIRE

(43) Date of publication of application: 06.11.2019
(73) Proprietor: Ficosa Adas, S.L.U., 08028 Barcelona (ES)
(72) Inventor: FERNANDEZ CHIMENO, Mireya, 08034 Barcelona (ES); RAMOS CASTRO, Juan, 08034 BArcelona (ES); GARCÍA GONZALEZ, Miguel Angel, 08034 Barcelona (ES); GUEDE FERNANDEZ, Federico, 08034 Barcelona (ES); MATEU MATEUS, Marc, 08034 Barcelona (ES); RODRIGUEZ IBAÑEZ, Noelia, E08232 Viladecavalls (ES); BAS PUJOLS, Bernat, E08232 Viladecavalls (ES); ALVAREZ GOMEZ, Jose Maria, E08232 Viladecavalls (ES)
(74) Representative: ZBM Patents - Zea, Barlocci & Markvardsen
(86) International application number: PCT/EP2016/082780
(87) International publication number: WO 2018/121861

(56) References cited:
- WO-A1-2014/128273
- WO-A2-2011/067788
- CN-A- 105 476 647
- US-A1- 2016 159 370
- US-A1- 2016 371 833

## Description

The present disclosure relates to methods and devices for respiratory signal extraction.

### BACKGROUND

Driving is a complex activity that involves correct perception, proper response time and adequate physical capacity, which can be compromised by the appearance of somnolence or drowsiness. In many cases the driver may not be completely aware of the entrance in such dangerous states. A somnolence detector may then be used. It has been shown that the analysis of the respiratory signal can give information related to drowsiness, especially concerning the variability of the respiratory patterns. However, most of the methods used to acquire respiration data are invasive or could distract or annoy the driver.

US2016/159370 discloses a driving assistance device.

CN105476647 relates to a method and equipment for helping a vehicle driver to breathe and a vehicle having the equipment.

WO2014/128273 describes one or more imaging device(s) inside a car that look(s) at occupants (driver, front passenger, rear passengers) and cover(s) multiple security, comfort, driver assistance and occupant state related functions, wherein the imaging device includes an illumination in the near infrared. Other relevant prior art is disclosed in US 2016/0371833 A1 and WO 2011/067788.

### SUMMARY

To overcome the drawbacks of the existing solutions, a non-invasive method is proposed without distracting the driver when driving. According to the proposed method the driver's chest or thoracic area is monitored using an imaging device such as an image sensor, i.e. a sensor that detects and conveys the information that constitutes an image, or a depth sensor, i.e. a device that may capture depth information, to measure movement and construct a respiratory signal from the measured movement.

In a first aspect, a method of extracting a respiratory signal is disclosed. The method comprises capturing a series of image frames of at least a portion of a driver's chest, generating an object signal from the series of image frames and constructing the respiratory signal from the generated object signals. The object signal may be associated to detected differences between image frames or to differences in the measured distance between the image capturing device and the thoracic area.

In some examples capturing the series of image frames may comprise using a remote sensing device such as an image sensor or a depth sensor. An image sensor may comprise any device for recording visual images in the form of photographs, film, or video signals. A depth sensor may comprise an infrared camera and an infrared laser projector to detect depth from the images or image frames captured.

In some examples, the method may further comprise identifying one or more objects on each captured image frame, extracting one or more features, respectively, of said one or more objects for each image frame. Generating an object signal from the series of image frames may then comprise generating an object signal from the respective extracted one or more features for each object and from the series of image frames.

When using a depth sensor the method may comprise analyzing changes in an axis (z) perpendicular to the image frames, produced by changes in the diaphragm of the driver due to the air flow during the inhalation and exhalation processes and the subsequent deformation of the thoracic cage. These changes in the perpendicular axis may be detected by the depth sensor.

The image capturing system may calculate a depth map from the distance between the sensor and the different objects in the sensor's field of vision. In some examples this map may be represented as a matrix of 16 bit integers. In order to minimize any noise produced by different environmental changes, such as background objects or objects passing in front of the depth sensor, a region of interest (ROI) may be established. To establish the ROI, an approximation of the distance between the driver and the depth sensor may be calculated in order to determine the approximate region (e.g. in a selected axis) in which the driver's movements may be maximized. Once the ROI is selected, depth vectors related to the ROI may be calculated and a mean of all the depth vectors inside the ROI may be computed in order to extract the respiratory signal.

When using an image sensor, identifying one or more objects may comprise identifying points or lines on the at least part of a portion of a driver's chest. For example, the points or lines may be part of the outline of the driver's chest.

In some examples, identifying one or more objects on each captured image frame may comprise identifying predetermined objects and matching those objects with reference objects. In other examples, the objects may not be predetermined and may be identified based on some inherent properties. For example, continuous lines of a minimum length or identifiable forms like closed or open curves on the chest or associated with the chest of the driver may be identified.

In some examples identifying predetermined objects may comprise identifying an object on a chest wearable item. In some examples the chest wearable item may be a seat belt. In other examples, the chest wearable item may be a chest garment, such as a t-shirt, a sweatshirt or the like. The garment may be selected to be tight enough so that the surface of the garment may move together with the respiration. The use of a predetermined object, i.e. an identifiable object by the image sensor, may improve the reliability of the system as the system may accurately identify control points on the predetermined objects.

In some examples, identifying one or more objects may comprise identifying a first set of lines or stripes of a first colour in one direction of a chest wearable item (e.g. on a T-shirt or on a seat belt). If more than one stripe is present in the set of stripes, then the stripes may be arranged in parallel. This allows for easier detection of the objects by the image processor.

In some examples identifying one or more lines or stripes may further comprise identifying a second set of lines or stripes of a second colour. The second set of stripes may be arranged at an angle, e.g. perpendicular, to the first set of stripes. This allows for a different channel to be applied by the image processor when extracting the objects.

In some examples, extracting one or more features of the one or more objects may comprise extracting a centroid of each of the one or more objects, respectively. This allows for a single point to be extracted from each object. The centroid may be then followed in time to generate an object signal. In other implementations more than one points or another point different than the centroid may be selected. In some examples the one or more features may also comprise the area of the objects identified. This may allow discarding objects that may not be useful for the respiratory signal extraction.

In some examples generating an object signal may comprise generating a space-time graph of the centroids of each object in the series of image frames. This allows for a consistent point to be followed in time that may correspond with the respiratory movement.

In some examples, constructing the respiratory signal may comprise generating a respiratory waveform by interpolating the centroids of the space-time graph. The interpolated waveform may correspond to the movement of the selected point in time. As the point movement may be attributed to the respiratory movement, assuming the driver is not moving in his/her chair, the interpolated waveform may be directly correlated with a respiratory waveform.

In some examples, identifying one or more objects may comprise identifying a set of unique patterns thus extracting a series of objects from the patterns.

In some examples, identifying those patterns may comprise identifying a series of features in the pattern given a reference image. These features may be sorted and filtered and an optical tracking of the extracted objects may be performed,
In some examples, generating an object signal may comprise calculating the position in time of the extracted objects, this position may be obtained from the extracted features of the pattern.

In some examples, generating the respiratory signal may comprise the evolution of the position in time of the extracted features, as the changes in the position may be attributed to the respiratory movement assuming the driver is seated. The given signal may be directly correlated with the respiratory waveform.

In another aspect, a device to extract a respiratory signal is disclosed. The device may comprise image frame capturing means to capture a series of image frames of at least a portion of a driver's chest. The device may further comprise image processing means. The image processing means may comprise object signal generation means, to generate an object signal from the series of image frames. The object signal generation means may be a video object extraction tool. The video object extraction tool may be a software tool. The device may further comprise respiratory signal generation means, to construct the respiratory signal from the generated object signals. The respiratory signal generation means may comprise signal filters, e.g. low-pass filters that may receive the object signal for each object and generate the respiratory signal.

The device may further comprise object identification means to identify one or more objects on each captured image frame. The image processing means may be a real-time image processing tool. The real-time image processing tool may be a software tool. The device may further comprise feature extraction means to extract one or more features of said one or more objects for each image frame, respectively. The feature extraction means may comprise an edge detector tool. The edge detector tool may be a software tool.

In yet another aspect, a computer program product is disclosed. The computer program product may comprise program instructions for causing a computing system to perform a method of extracting a respiratory signal according to some examples disclosed herein.

The computer program product may be embodied on a storage medium (for example, a CD-ROM, a DVD, a USB drive, on a computer memory or on a read-only memory) or carried on a carrier signal (for example, on an electrical or optical carrier signal).

The computer program may be in the form of source code, object code, a code intermediate source and object code such as in partially compiled form, or in any other form suitable for use in the implementation of the processes. The carrier may be any entity or device capable of carrying the computer program.

For example, the carrier may comprise a storage medium, such as a ROM, for example a CD ROM or a semiconductor ROM, or a magnetic recording medium, for example a hard disk. Further, the carrier may be a transmissible carrier such as an electrical or optical signal, which may be conveyed via electrical or optical cable or by radio or other means.

When the computer program is embodied in a signal that may be conveyed directly by a cable or other device or means, the carrier may be constituted by such cable or other device or means.

Alternatively, the carrier may be an integrated circuit in which the computer program is embedded, the integrated circuit being adapted for performing, or for use in the performance of, the relevant methods.

The invention is defined in the independent claims.

### BRIEF DESCRIPTION OF THE DRAWINGS

Non-limiting examples of the present disclosure will be described in the following, with reference to the appended drawings, in which:
Figure 1 schematically illustrates a device to extract a respiratory signal according to an example;
Figure 1A schematically illustrates a flowchart of a respiratory signal extraction algorithm according to an example;
Fig. 2A, 2B and 2C schematically illustrate drivers with chest wearable items;
Figures 3A to 3H and Figures 4A to 4D schematically illustrate an image frame of a driver wearing a chest wearable item at various processing stages;
Figure 5 illustrates an example plot of objects detected at a series of image frames;
Figure 6 shows the magnitude response of a filter for an extracted respiratory signal.
Figure 7 is a flow chart of a method of extracting a respiratory signal according to another example.
Figure 8 is a flow chart of a method of extracting a respiratory signal according to yet another example.

### DETAILED DESCRIPTION OF EXAMPLES

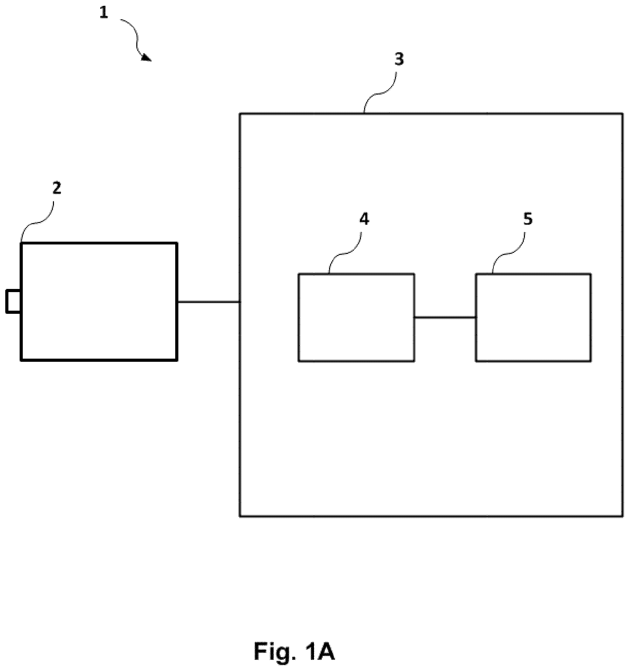

Figure 1A schematically illustrates a device to extract a respiratory signal according to an example. Device 1 may comprise image capturing means 2 and image processing means 3. The image capturing means may comprise a depth sensor. The depth sensor may comprise an infrared camera and an infrared laser projector to detect depth from the image capturing. For example, the infrared camera of the depth sensor may have a VGA (640x480) resolution at 200 fps with a working range of approximately 20 cm up to 150 cm. The depth sensor may produce a matrix of e.g. 16 bit integers that may represent the distance in meters between the depth sensor and the portion of the chest. The image processing means may comprise a depth map calculator 4 and a respiratory signal generation tool 5. The depth map calculator may calculate the distance between the depth sensor 2 and the different objects in the sensor's field of vision. The depth map calculator 4 represent images captured as a matrix of 16 bit integers. In order to minimize any noise produced by different environmental changes, such as background objects or objects passing in front of the depth sensor, the depth map calculator may establish a region of interest (ROI) within which any identified points may contain the information required to reconstruct a respiratory signal. To establish the ROI, the depth map calculator 4 may calculate an approximation of the distance between the driver and the depth sensor in order to determine the approximate region (e.g. in the axis perpendicular to the image frames) in which the driver's movements may be maximized. Once the ROI is selected, the respiratory signal generator tool 5 may compute a mean of all the points inside the ROI in order to extract the respiratory signal.

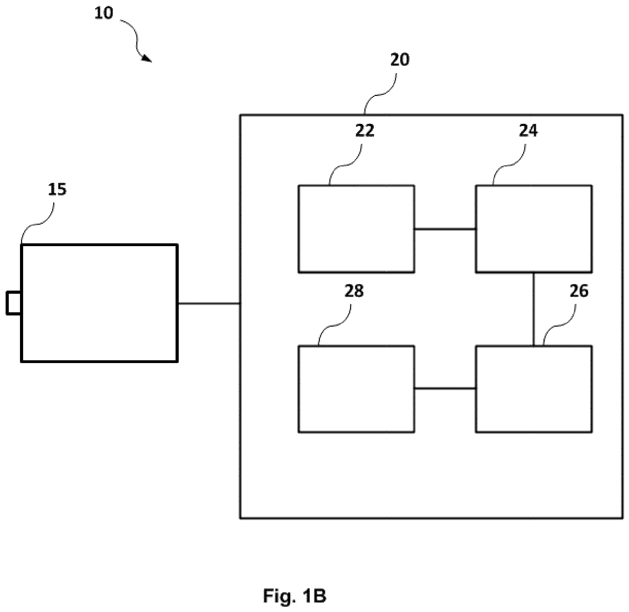

Figure 1B schematically illustrates a device to extract a respiratory signal according to another example. Device 10 may comprise an image sensor 15 and image processing means 20. The image sensor may capture series of image frames of a chest wearable item of a driver, e.g. seat belt or garment, and transfer the image frames to the image processing means 20. The image sensor 15 may be a video camera with a full HD sensor of 1080x1920 at 30 frames-per-second (fps). The video camera may produce frames in RGB at 8 bit integers per channel. The video camera 15 may be connected to the image processing means 20. Alternatively, the video camera 15 may comprise a communication interface and transmit the captured image frames to the image processing means 20. The image processing means 20 may be a software application that may process real-time images. The software application may comprise a series of blocks, tools or modules that may be synchronised to extract the respiratory signal from the captured series of images. The tools may comprise an object identification tool 22, a features extraction tool 24, an object signal generation tool 26 and a signal generation tool 28.

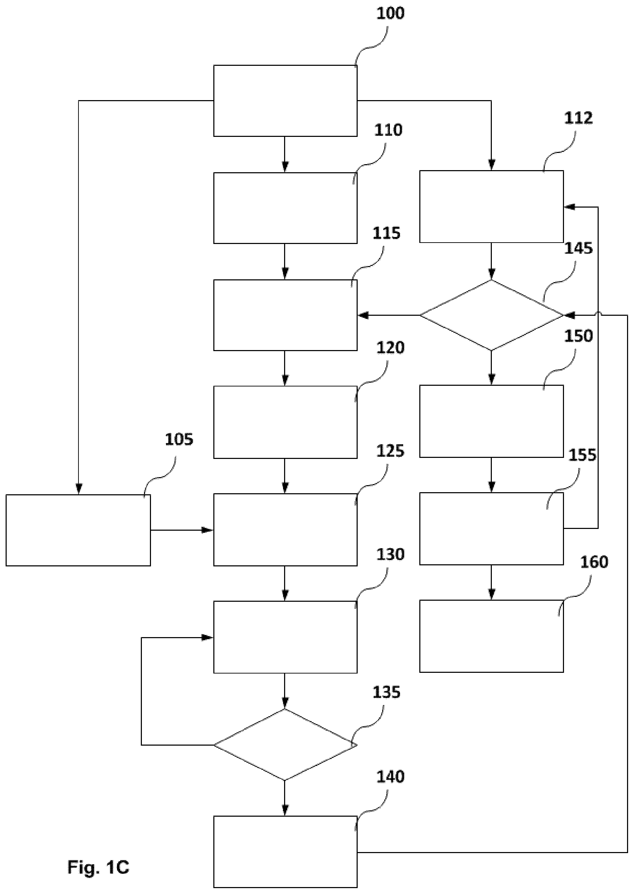

Fig. 1C schematically illustrates a flowchart of a respiratory signal extraction algorithm according to an example. In block 100 the process may be initialised. In block 105 the number of patterns to detect may be defined. In block 110 a reference image is acquired. In block 112 a new image frame is provided. If the new frame is the first frame then the image may be processed by block 115 and a number of features of the first image frame may be extracted by block 115. In block 145 it may be checked if there are enough features in the new frame that has been provided. If not enough features are present, then the process continues to block 115. In block 115 a number of features may be extracted from the reference image and from the new frame that has been provided. For example, an ORB detector may be used. Block 115 may then provide reference features and frame features. In case an ORB detector is used, block 115 may provide descriptors and keypoints for the reference image and the captured frame. In block 120 the extracted features may be matched. For example, block 120 may use Flann matching to provide a number of matched features. Then in block 125 the matched features may be clusterised based on the number of patterns to detect defined in block 105. Thus, the number of patterns may correspond to the number of clusters to be generated by the clusterization block 125. For example, k-means clustering may be used to provide the defined number of clusters, each having k points. The clustered features may then be provided to block 130 where a homography transformation, e.g. Ransac Homography, may take place for each cluster. Block 135 checks if all clusters have been transformed by the homography block 130. When all clusters are transformed, then the features or points to track may be identified in block 140. The process then continues to block 145 where it is checked again if there are enough features. If the answer is negative, then the process continues as discussed above in block 115. If the answer is positive, then the process continues to block 150, where the features are tracked. That is, the features may be associated to features belonging to previous frames. For example, a Lucas-Kanade Optical Flow tracking method may be used. After the features are tracked in block 150, a signal may be extracted in block 155. For example, the mean of the modulus for each point or feature tracked may be calculated. Then, based on the extracted signal, the raw respiratory signal may be reconstructed in block 160. To reconstruct the raw signal, the extracted signal may be filtered and conditioned.

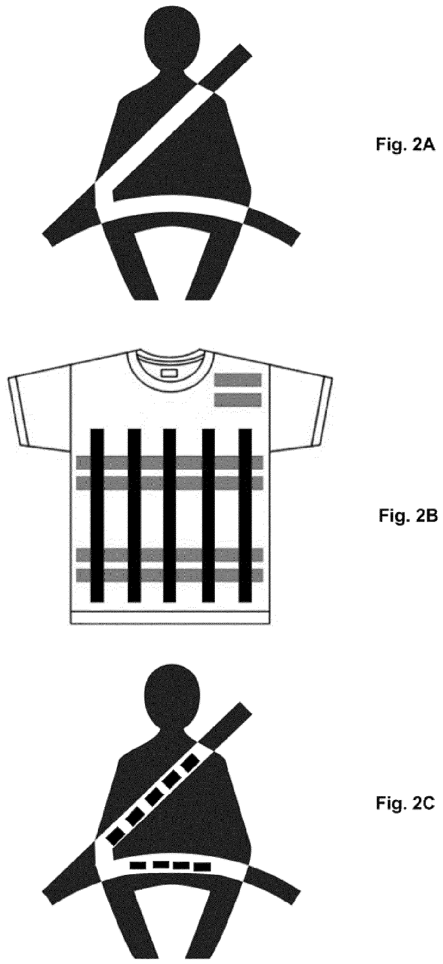

Fig. 2A, 2B and 2C schematically illustrate drivers with chest wearable items. In the example of Fig. 2A the chest wearable item is a seat belt. The seat belt may be a three-point seat belt, thus defining two stripes arranged at an angle. One stripe may be traversing the chest of the driver. This stripe may be the one used to extract the respiratory signal of the driver. In the example of Fig. 2B the chest wearable item is a garment such as a T-shirt. The T-shirt may comprise a white background, and rectangular stripes. The stripes may be of different colours to facilitate features extraction. For example, the vertical stripes may be painted in black and placed along the T-shirt width, and horizontal stripes may be painted in red, and placed in the shoulders, thorax and abdomen. In the example of Fig. 2C the chest wearable item may be a pattern or patterns embedded into the seat belt. The pattern or patterns may be used to extract the respiratory signal of the driver.

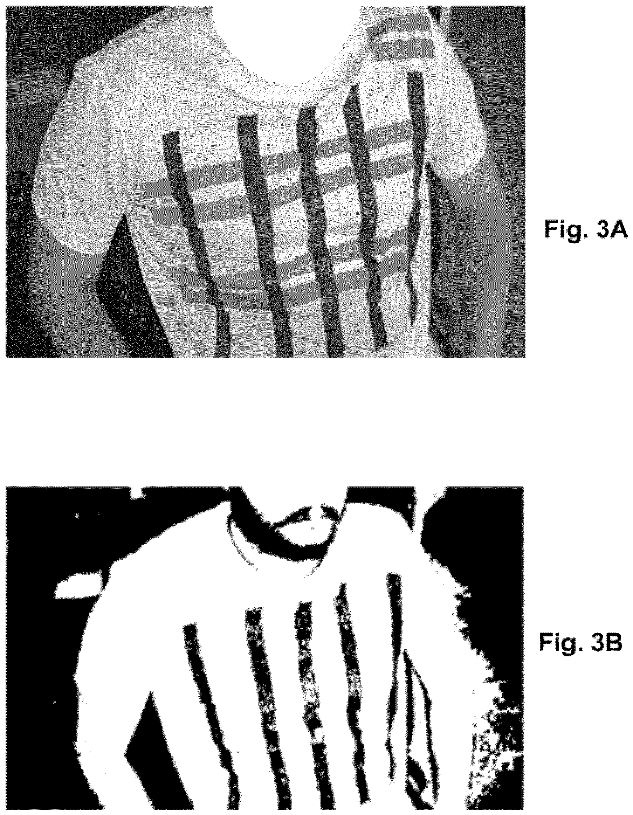

The respiratory signal extraction method shall be described with the example of the T-shirt for object identification. A driver wearing the T-shirt may be seated at the driver seat. Then the image sensor may start recording image frames of the T-shirt. Fig. 3A shows an image frame as captured by the image sensor. The image sensor may be placed at the interior rear-view mirror of the vehicle or behind the steering wheel. Alternatively, the image sensor may be placed at a lateral position. Then any patterns on the chest wearable item may be identified using the silhouette or the frame of the body of the driver or other patterns on the driver or in the vehicle. In the example of Fig. 3A the image sensor was placed at the interior rear-view mirror. In other examples more than one image sensor may be used. The algorithm performed by the image processing tool may be divided in four steps: a) object extraction, b) feature extraction, c) object signal generation, d) respiratory signal construction.

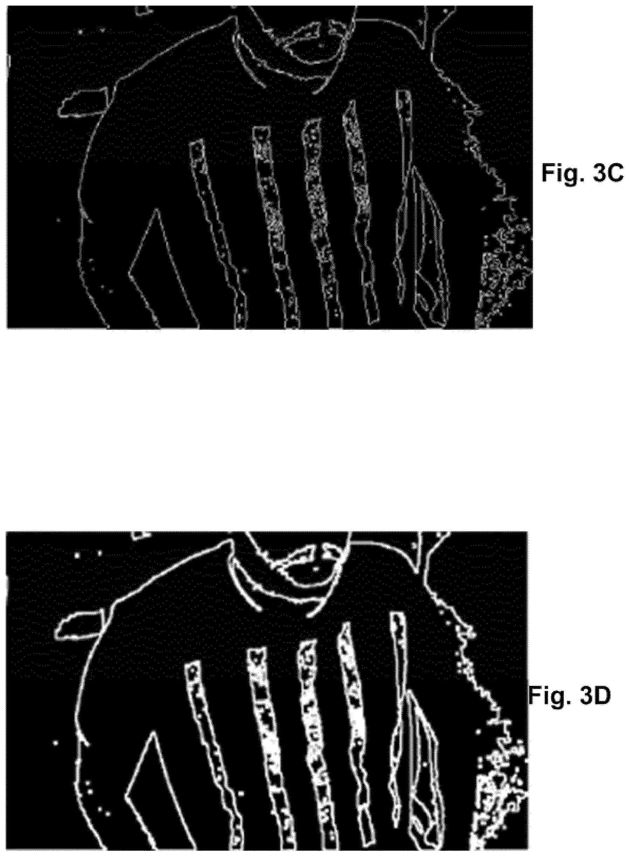

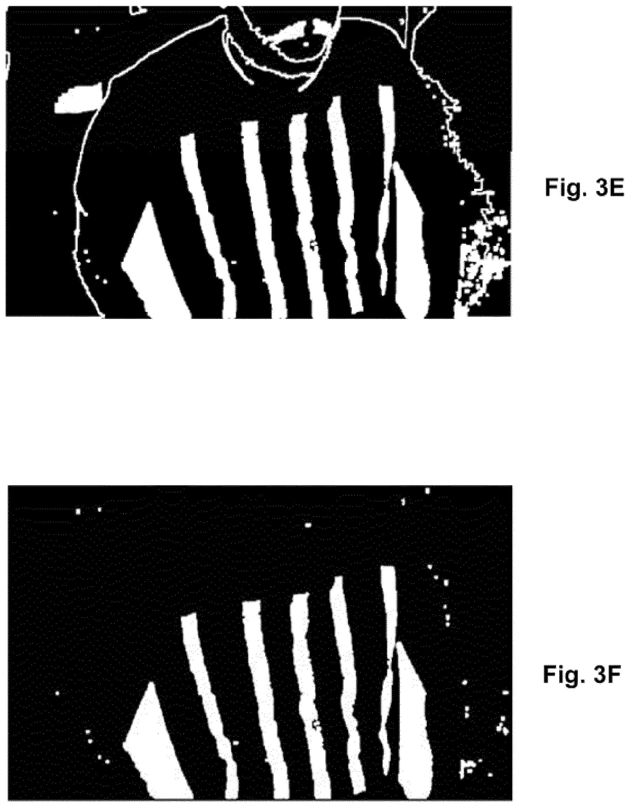

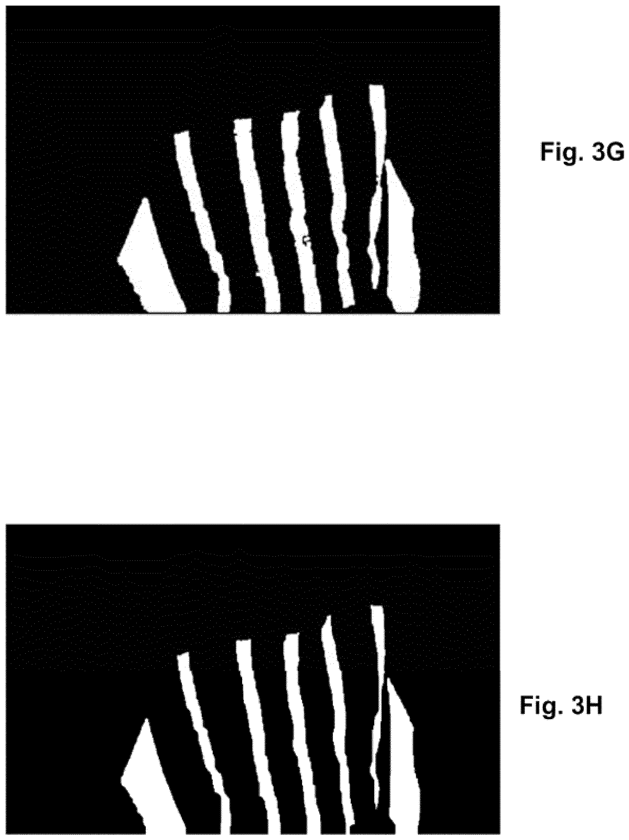

During object extraction, all the objects for each single frame or image may be detected. Since it is known that the objects on the T-shirt are red and black, a specific processing based on filtering these colours may be performed. A different treatment may be performed for red than for black objects. An example of a grayscale video frame is presented in Fig. 3A. Then the frame may be binarized with a certain threshold optimized in order to maximize the black object detection and minimize the other pixels detection. Fig. 3B is the binarized image of Fig. 3A. Then, a high-pass filter, e.g. a Sobel filter, may be applied in order to extract the borders of the image, with a given threshold computed automatically for every frame. If the binarization is perfect and only the desired objects were extracted, the high-pass filter may give the edges of the T-shirt objects. However, as several other pixels may be taken in the binarization step, it may be needed to distinguish the objects of interest from the other pixels, noise, etc. The filtered frame is shown in Fig. 3C. As a result, the edges are extracted. In some cases the borders may not be compact and they may have holes in the object perimeter. In order to fix that, a dilatation of the image may be performed, with a squared structural element of an amount of pixels proportional to the image size. After this step, the objects' edges may compactly surround the objects. Once the objects are well surrounded, a function to fill the holes may be applied, so that the whole object is detected. Fig. 3D shows the dilated image and Fig. 3E shows the image with the holed filled. After these steps, the objects may be well extracted, but many other pixel groups may also be extracted. Therefore a processing to clean these other pixel groups may be performed. For that purpose, firstly all the border objects may be removed, assuming that the useful objects may be located in the center of the image. Then, a minimum object size proportional to the image size may be defined, and all the objects smaller than this size may be cleared. Thus, all the non-desired objects may be cleared. Fig. 3F shows the image with the border objects removed and Fig. 3G shows the image with the small-objects cleared.

Then, the dilation step previously done has to be undone, by smoothing the image with the same structural element. Finally, an opening is applied to the image in order to make the object borders smoother. The final image is shown in Fig. 3H.

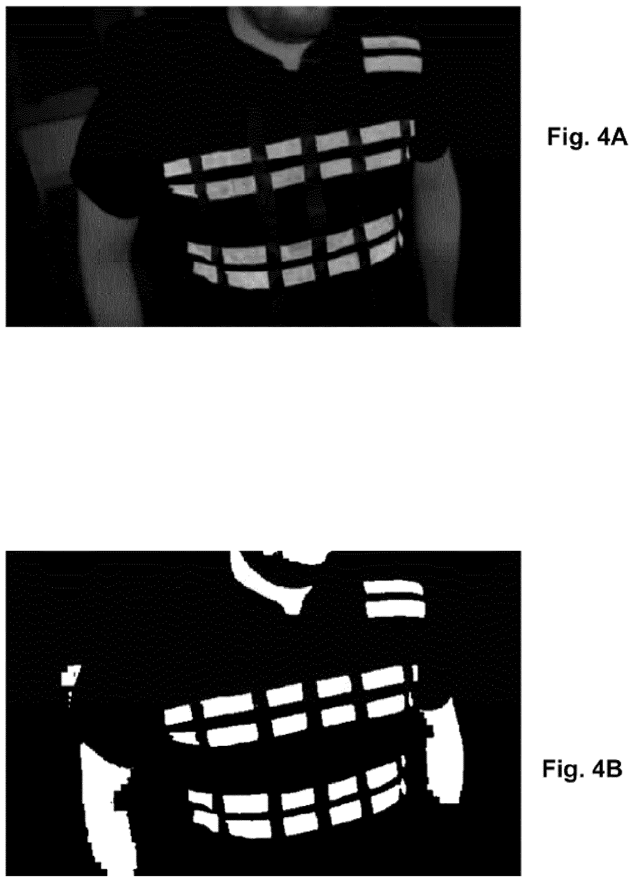

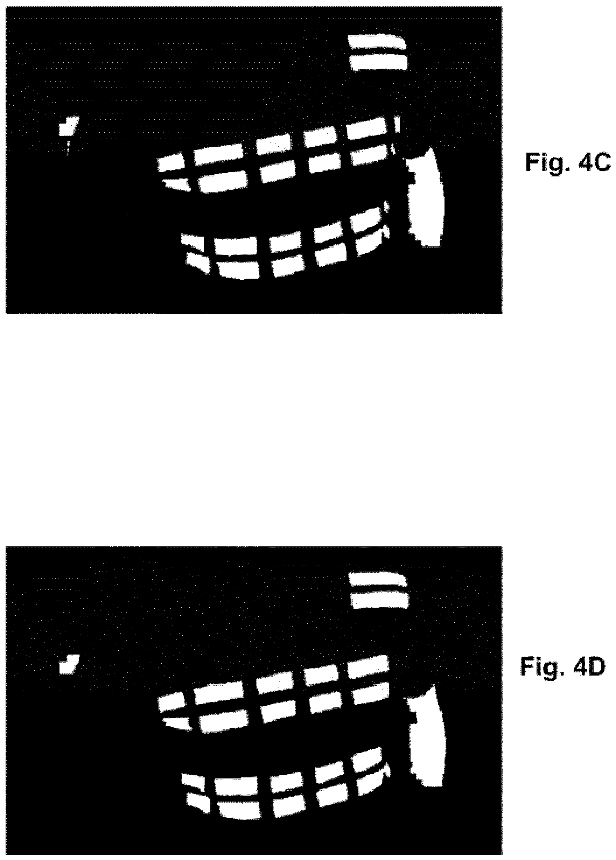

A similar process may be applied for the red objects, but with some small differences. Firstly, the RGB image may be converted into a single matrix by taking the red channel and subtracting the blue component. This process may give the highest contrast between red color and other colors. The result is shown in Fig. 4A. Taking this image as input frame, the same binarization function may be used for the red objects as the one used for the black objects, but with a different threshold, also optimized for the red object detection purpose. As it can be seen in Fig. 4B, apart from the useful objects many other pixels are detected. These need to be removed. The same steps that were applied for black objects may be applied in the image of Fig. 4B, in order to clear any non-desired objects. Thus, all the objects smaller than a certain size as well as all the border objects may be removed. Fig. 4C shows the border-objects removed and Fig. 4D shows the small objects cleared.

If any undesired objects remain, e.g. the arm, then they may be removed by analysing their appearance along the video, as the T-shirt objects may remain in about the same position and the same size while undesired objects may change their size or position, e.g., every time the subject moves the arm.

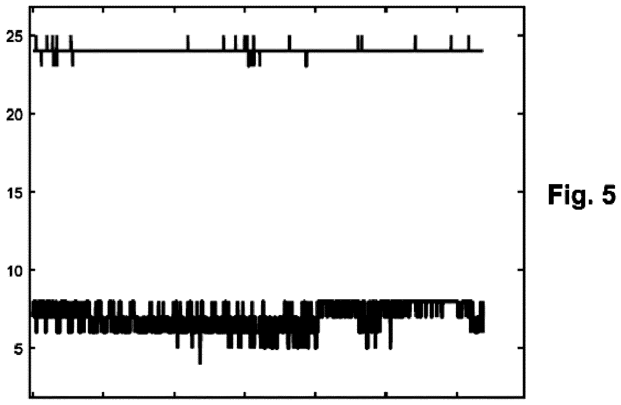

The number of objects detected at each frame may be saved, either at an internal memory of the system or at a remote memory location, and/or displayed at a local or remote system monitor, so it can be easily seen whether some objects appear or disappear. An example plot of the objects is shown in Fig. 5. It can be seen that there may be an offset of about two objects in both red and black cases.

Once all the objects are identified, it is now possible to extract features from them that may be used for the respiratory signal extraction. Furthermore, feature extraction may help clear any wrongfully identified objects.

For the respiratory signal extraction, a feature related with the object's position may be used. Some examples may include the top pixel, the bottom pixel, the bottom trend line (the line that approaches the best the bottom edge of the object), the mean of the left-right pixels for the horizontal position and the mean of the top-bottom for the vertical position or the centroid of the objects. The centroid (also called center of gravity or geometrical center) may have the advantage that it is both easy to extract and useful for the respiration extraction. Thus, for every object in every frame the calculus of the centroid may be performed. Additionally, the object's area may also be computed. All this information may then be used to discard any undesired objects. In the example of Fig. 3A to 3H, it is expected that objects that were not supposed to be taken as T-shirt objects (e.g. background, arms, shadow etc.) may change their area and center faster than any useful T-shirt objects would. Also, some valid T-shirt objects may be partially or totally occluded during the image sequence or video capturing, and therefore may not be used in the respiration extraction if they do not appear in a minimum amount of frames or images. If the image sensor is placed next to the interior rear-view mirror, and taking into account the curvature of the body, some objects to the left of the subject may be sometimes partially occluded. These objects may be at times appearing and disappearing, or merging between them due to e.g. T-shirt creases, wrinkles or folds. If two objects may merge, their center and area may suddenly change from image to image and will thereby be detected and discarded in further steps.

After extracting objects at frame or image level, a new iteration may be performed in order to find the continuity of these objects. The first frame's objects' position may be selected and, frame by frame, all the object positions may be compared with the previous frame's object positions. If a position coincides, with a predetermined offset, with any of the previous frame's object position, it may be assumed that it is part of the same object and may be added in an object column. If no such coincidence may be found, it may be assumed as a new object and placed in a new column. Thus a matrix may be created, in which each column may represent an object, and each row may represent each frame. Then, each object's area may also be checked, and if a change is suddenly detected, then this may be flagged as an object irregularity, and the object may be tagged as doubtful.

The next step is to interpolate the signal for each extracted feature. An initial time may be set, a new time line may be created for the video signal, and every object position signal may be interpolated in order to, firstly have a first sampling rate (e.g. of 40 samples per second) and secondly fill any empty values along the object position, if, for example, in some frames the object was occluded, or some frames were declared as invalid, so they do not have data.

The last step is to compute the respiratory signal. All the detected objects may be classified into one of the following groups: shoulders, thorax, abdomen or vertical objects. Each object may be labelled with one of the mentioned groups, based on its position relative to the other objects. Once each object has been labelled, for each pair of objects a respiratory signal may be computed. The respiratory signals may be grouped depending on the labelling of the two objects from which it has been extracted.

The respiratory signals may be labelled as one of the following:
- Vertical (both objects may be "vertical")
- Shoulders (both objects may be "shoulders")
- Thorax (both objects may be "thorax")
- Abdomen (both objects may be "abdomen")
- Shoulders-Thorax ("shoulders" and "thorax" objects)
- Shoulders-Abdomen ("shoulders" and "abdomen" objects)
- Thorax-Abdomen ("thorax" and "abdomen" objects)

And this may be done for all the possible object combinations, having several respiratory signals with each label, having many different possibilities within each group.

Another respiratory signal extraction method could be explained with the example of the pattern detection. The seat belt may contain a given pattern embedded into its fabric. The image sensor may be placed facing the subject and capture a sequence of image frames. The algorithm performed by the image processing tool may be divided in two states: a) Not enough detected features to track and b) Enough detected features to track. These two states may be divided into further steps: a1) Feature extraction, a2) Feature matching, a3) Clustering, a4) Checking detected features to track, b1) Object tracking and b2) Respiration extraction.

If the image frame is the first one or the features are not enough, the algorithm may extract all the features of a given reference image from the used pattern and of the received image frame. Once the features are extracted, the algorithm may perform a matching of all the features from the reference image and the given captured image frame. The algorithm may perform a clustering technique to detect multiple patterns. The algorithm may perform a Ransac homography detection over the matched features for each one of the clusters and it may detect all the bijective features. The algorithm may include all the resulting features to a feature pool.

If the number of features is enough, the algorithm may calculate the new position of the previous features by the means of optical flow algorithms ex: Lucas-Kanade, and it may discard the features that present a deviation greater than a given constant. The algorithm may calculate the modulus for each one of the new features. The algorithm may calculate the mean of the previous modulus. The algorithm may reconstruct the respiration signal from the previous mean.

The algorithm has an iterative nature so it may be executed once for each image frame, and it may decide which part is executed at any given time.

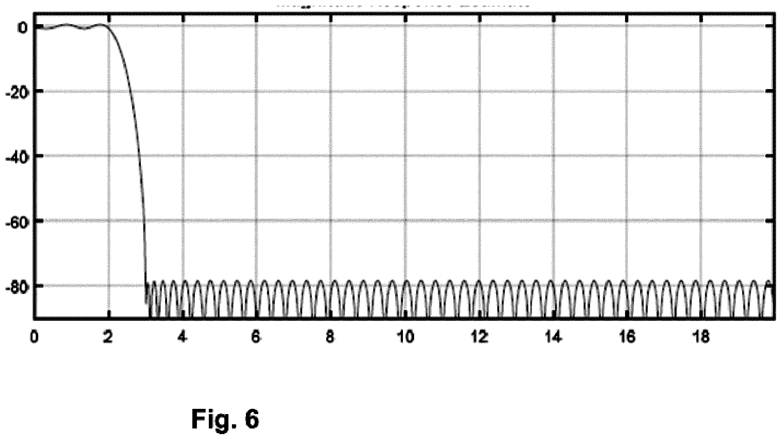

It is known that a respiratory signal may be a very low frequency signal, usually below 0,5Hz. The typical respiratory rate for a healthy adult at rest is 12-20 breaths per minute (each respiratory cycle lasts for more than two second). These values are typical values at rest, but when the subject is doing exercise, even light, this may increase significantly. For that reason, the extracted signals may be filtered, in order to reduce the noise and the leave only the low frequency oscillations (that is, the ones related with the respiration). Although a usual driving does not imply an effort that could significantly increase the respiratory rate, it may be taken into account that in some cases it could happen. Therefore the filter pass-band may be up to 2Hz, and the stop-band may be up to 2,5Hz. This means that while the respiratory rate remains below 2Hz (i.e. 120 cycles per minute, much higher than the typical respiratory rate), the signal may not be affected, i.e. the filter magnitude response may not be completely flat. All the oscillations above 2,5Hz may be significantly reduced. Fig. 6 shows the magnitude response of the designed filter.

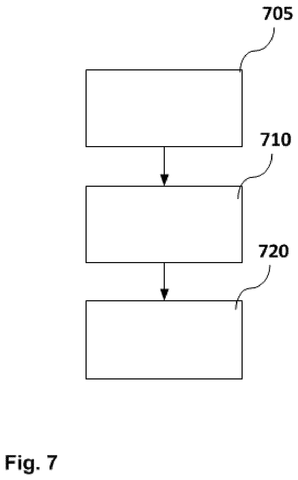

Fig. 7 is a flow chart of a method of extracting a respiratory signal according to another example. In block 705 a series of image frames of at least a portion of a driver's chest may be captured by an image capturing device, e.g. an image sensor or a depth sensor. In block 710, an object signal may be generated from the captured series of image frames. Then, in block 720, the respiratory signal may be constructed from the generated object signals.

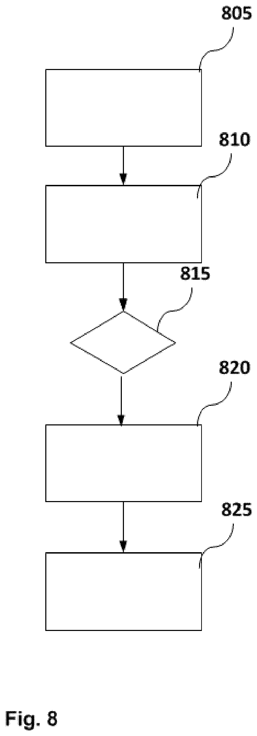

Fig. 8 is a flow chart of a method of extracting a respiratory signal according to yet another example. In block 805 a series of image frames of at least a portion of a driver's chest may be captured by a depth sensor. Then, in block 810, a depth map or matrix may be calculated based on the distance between the depth sensor and the different objects in the sensor's field of view. In block 815, it is checked if a point in the calculated depth map belongs to a region of interest. If a point does not belong to the ROI it may be excluded from further calculations. Then, in block 820, a mean value of all the points inside the region of interest may be computed for each frame. Finally, in block 825, the respiratory signal may be constructed for all the image frames and for all the point mean values.

Although only a number of examples have been disclosed herein, other alternatives, modifications, uses and/or equivalents thereof are possible. Thus, the scope of the present invention should not be limited by particular examples, but is determined only by the claims that follow.

Further, although the examples described with reference to the drawings comprise computing apparatus/systems and processes performed in computing apparatus/systems, the disclosure also extends to computer programs, particularly computer programs on or in a carrier, adapted for putting the system into practice.

## Claims

1. A method of extracting a respiratory signal, comprising:
capturing a series of image frames of at least a portion of a driver's chest, wherein capturing a series of image frames comprises using an image sensor (15) to capture the series of image frames;
identifying one or more objects on each captured image frame, wherein identifying one or more objects comprises:
performing a binarization of each captured image frame with a threshold;
applying a high-pass filter to each captured image frame in order to extract borders of the corresponding image frame;
performing a dilation of each captured image frame such that the borders of the corresponding image frame are compacted;
removing objects of the borders of each captured image frame;
undoing the dilation of each captured image frame by smoothing the corresponding image frame with a same structural element;
applying an opening to each captured image frame;
extracting one or more features, respectively, of said one or more objects for each image frame;
generating an object signal from the series of image frames, wherein generating an object signal from the series of image frames comprises generating an object signal from the respective extracted one or more features for each object and from the series of image frames;
constructing the respiratory signal from the generated object signals.

2. The method according to claim 1, further comprising after removing objects from the border of each image frame,defining a minimum object size proportional to each captured image frame size;
clearing the objects of each captured image frame smaller than the defined minimum object size.

3. The method according to claim 1, wherein identifying one or more objects comprises identifying points or lines on the at least part of a portion of a driver's chest.

4. The method according to claim 1, wherein identifying one or more objects on each captured image frame comprises identifying predetermined objects and matching those objects with reference objects.

5. The method according to any of claims 1 or 3 - 4, wherein extracting one or more features of said one or more objects comprises extracting a centroid of each of the one or more objects, respectively.

6. The method according to any of claims 1 or 3 - 5, wherein identifying one or more objects comprises identifying a set of unique patterns.

7. The method according to claim 6, wherein identifying a unique pattern comprises extracting and matching features from a reference image and the captured image frames.

8. The method according to any of claims 6 to 7, further comprising tracking of the extracted and matched features by means of an optical flow algorithm.

9. The method according to claim 8, wherein tracking of the extracted and matched features comprises calculating the modulus for each tracked and matched feature.

10. A device (10) to extract a respiratory signal, comprising:
Image frame capturing means (2) to capture a series of image frames of at least a portion of a driver's chest, wherein the image frame capturing means comprises an image sensor (15);
object identification means to identify one or more objects on each captured image frame, where identifying one or more objects comprises:
performing a binarization of each captured image frame with a threshold;
applying a high-pass filter to each captured image frame in order to extract borders of the corresponding image frame;
performing a dilation of each captured image frame such that the borders of the corresponding image frame are compacted;
removing objects of the borders of each captured image frame;
undoing the dilation of each captured image frame by smoothing the corresponding image frame with a same structural element;
applying an opening to each captured image frame;feature extraction means to extract one or more features of said one or more objects for each image frame, respectively;
Image processing means (3), the image processing means (3) comprising:
object signal generation means, to generate an object signal from the series of image frames, wherein the object signal generation means is configured to generate the object signal from the respective extracted one or more features for each object and from the series of image frames.
respiratory signal construction means, to construct the respiratory signal from the generated object signals.

11. The device according to claim 10, further comprising:
means for defining a minimum object size proportional to each captured image frame size, and
means for clearing the objects of each captured image frame smaller than the minimum object size.

12. The device according to claim 10, wherein the image processing means comprises a real-time image processing engine.

13. The device according to claim 10, wherein the object extraction means comprises a centroid calculator.

14. The device according to any of claims 10 or 13, wherein the object signal generation means comprises a distance calculator to identify pairs of features belonging to the same object in successively captured image frames.

15. The device according to any of claims 10 or 13 - 14, wherein the signal generation means comprises a low-pass filter to receive the object signal for each object and generate the respiratory signal.

## Patentansprüche

1. Ein Verfahren zum Extrahieren eines Atmungssignals, umfassend:
erfassen einer Reihe von Bildfeldern von mindestens einem Teil der Brust eines Fahrers, wobei das Erfassen einer Reihe von Bildfeldern das Verwenden eines Bildsensors (15) zum Erfassen der Reihe von Bildfeldern umfasst;
identifizieren von einem oder mehreren Objekten auf jedem erfassten Bildfeld, wobei das Identifizieren von einem oder mehreren Objekten Folgendes umfasst:
durchführen einer Binärisierung von jedem erfassten Bildfelds mit einem Schwellenwert;
anwenden eines Hochpassfilters auf jeden erfassten Bildfeld, um Ränder des entsprechenden Bildfelds zu extrahieren;
durchführen einer Aufweitung von jedem erfassten Bildfeld, so dass die Ränder des entsprechenden Bildfelds verdichtet werden;
entfernen von Objekten der Ränder von jedem erfassten Bildfeld;
rückgängigmachen der Aufweitung von jedem erfassten Bildfeld durch Glätten des entsprechenden Bildfelds mit demselben Strukturelement;
anwenden einer Öffnung auf jedes erfasstes Bildfeld;
extrahieren eines oder mehrerer Merkmale jeweils von dem einen oder den mehreren Objekten für jedes Bildfeld;
erzeugen eines Objektsignals aus der Reihe von Bildfeldern, wobei das Erzeugen eines Objektsignals aus der Reihe von Bildfeldern das Erzeugen eines Objektsignals aus dem bzw. den jeweiligen extrahierten einen bzw. den mehreren Merkmalen für jedes Objekt und aus der Reihe von Bildfeldern umfasst;
konstruieren des Atmungssignals aus den erzeugten Objektsignalen.

2. Das Verfahren nach Anspruch 1, ferner umfassend, nach dem Entfernen von Objekten von dem Rand jedes Bildfelds, Definieren einer minimalen Objektgröße proportional zu jeder erfassten Bildfeldgröße;
löschen der Objekte von jedem erfassten Bildfeld, die kleiner als die definierte minimale Objektgröße sind.

3. Das Verfahren nach Anspruch 1, wobei das Identifizieren von einem oder mehreren Objekte das Identifizieren von Punkten oder Linien auf dem mindestens einen Teil eines Abschnitts der Brust eines Fahrers umfasst.

4. Das Verfahren nach Anspruch 1, wobei das Identifizieren von einem oder mehreren Objekten auf jedem erfassten Bildfeld das Identifizieren von vorbestimmten Objekten und das Zuordnen von diesen Objekten zu Referenzobjekten umfasst.

5. Das Verfahren nach einem der Ansprüche 1 oder 3 bis 4, wobei das Extrahieren von einem oder von mehreren Merkmalen des einen oder der mehreren Objekte jeweils das Extrahieren eines Schwerpunkts von jeden des einen oder der mehreren Objekte umfasst.

6. Das Verfahren nach einem der Ansprüche 1 oder 3 bis 5, wobei das Identifizieren von einem oder von mehreren Objekten das Identifizieren einer Reihe von eindeutigen Mustern umfasst.

7. Das Verfahren nach Anspruch 6, wobei das Identifizieren eines einzigartigen Musters das Extrahieren und Zuordnen von Merkmalen aus einem Referenzbild und den erfassten Bildfeldern umfasst.

8. Das Verfahren nach einem der Ansprüche 6 bis 7, ferner umfassend das Verfolgen der extrahierten und zugeordneten Merkmale mittels eines optischen Flussalgorithmus.

9. Das Verfahren nach Anspruch 8, wobei das Verfolgen der extrahierten und zugeordneten Merkmale das Berechnen des Moduls für jedes verfolgte und zugeordnete Merkmal umfasst.

10. Eine Vorrichtung (10) zum Extrahieren eines Atmungssignals, umfassend:
Ein Mittel zur Erfassung von Bildfeldern (2) zum Erfassen einer Reihe von Bildfeldern von mindestens einem Abschnitt der Brust eines Fahrers, wobei das Mittel zur Erfassung von Bildfeldern einen Bildsensor (15) umfasst;
ein Mittel zur Identifikation von Objekten zum Identifizieren von einem oder mehreren Objekten auf jedem erfassten Bildfeld, wobei das Identifizieren von einem oder mehreren Objekten Folgendes umfasst:
durchführen einer Binärisierung von jedem erfassten Bildfeld mit einem Schwellenwert;
anwenden eines Hochpassfilters auf jedes erfasste Bildfeld, um Ränder des entsprechenden Bildfelds zu extrahieren;
durchführen einer Aufweitung von jedem erfassten Bildfeld, so dass die Ränder des entsprechenden Bildfelds verdichtet werden;
entfernen von Objekten der Ränder von jedem erfassten Bildfeld;
rückgängigmachen der Aufweitung von jedem erfassten Bildfeld durch Glätten des entsprechenden Bildfelds mit demselben Strukturelement;
anwenden einer Öffnung auf jeden erfassten Bildfeld; ein Mittel zur Extraktion von Merkmalen, um jeweils ein oder mehrere Merkmale des einen oder der mehreren Objekte für jedes Bildfeld zu extrahieren;
Ein Mittel zur Verarbeitung von Bildern (3), wobei das Mittel zur Verarbeitung von Bildern (3) Folgendes umfasst:
Ein Mittel zur Erzeugung von Objektsignalen, um ein Objektsignal aus der Reihe von Bildfeldern zu erzeugen, wobei das Mittel zur Erzeugung von Objektsignalen konfiguriert ist, um das Objektsignal aus dem jeweiligen extrahierten einen oder mehreren Merkmalen für jedes Objekt und aus der Reihe von Bildfeldern zu erzeugen.
Ein Mittel zur Konstruktion von Atemsignalen, um das Atemsignal aus den erzeugten Objektsignalen zu konstruieren.

11. Die Vorrichtung nach Anspruch 10, weiter umfassend:
ein Mittel zum Definieren von einer minimalen Objektgröße, die proportional zu jeder erfassten Bildfeldgröße ist, und
ein Mittel zum Löschen der Objekte von jedem erfassten Bildfeld, das kleiner als die minimale Objektgröße ist.

12. Die Vorrichtung nach Anspruch 10, wobei das Mittel zur Verarbeitung von Bildern eine Echtzeitbildverarbeitungsmaschine umfasst.

13. Die Vorrichtung gemäß Anspruch 10, wobei das Mittel zur Extraktion von Objekten einen Schwerpunktrechner aufweist.

14. Die Vorrichtung nach einem der Ansprüche 10 oder 13, wobei das Mittel zur Erzeugung von Objektsignalen einen Abstandsrechner umfasst, um Paare von Merkmalen zu identifizieren, die zu demselben Objekt in aufeinanderfolgend erfassten Bildfeldern gehören.

15. Die Vorrichtung nach einem der Ansprüche 10 oder 13 bis 14, wobei das Mittel zur Erzeugung von Signalen ein Tiefpassfilter umfasst, um das Objektsignal für jedes Objekt zu empfangen und das Atmungssignal zu erzeugen.

## Revendications

1. Un procédé d'extraction d'un signal respiratoire, comprenant :
capturer une série de cadres d'image d'au moins une partie de la poitrine d'un conducteur, dans lequel la capture d'une série de cadres d'image comprend l'utilisation d'un capteur d'image (15) pour capturer la série de cadres d'image ;
identifier un ou plusieurs objets sur chaque cadre d'image capturé, dans lequel l'identification d'un ou plusieurs objets comprend :
effectuer une binarisation de chaque cadre d'image capturé avec un seuil ;
appliquer un filtre passe-haut à chaque cadre d'image capturé afin d'extraire des bordures du cadre d'image correspondant ;
effectuer une dilatation de chaque cadre d'image capturé de sorte que les bordures du cadre d'image correspondant soient compactées ;
retirer des objets des bordures de chaque cadre d'image capturé ;
annuler la dilatation de chaque cadre d'image capturé en lissant le cadre d'image correspondant avec un même élément structurel ;
appliquer une ouverture à chaque cadre d'image capturé ;
extraire une ou plusieurs caractéristiques, respectivement, desdits un ou plusieurs objets pour chaque cadre d'image ;
générer un signal d'objet à partir de la série de cadres d'image, dans lequel la génération d'un signal d'objet à partir de la série de cadres d'image comprend la génération d'un signal d'objet à partir de l'une ou des plusieurs caractéristiques extraites respectives pour chaque objet et à partir de la série de cadres d'image ;
construire le signal respiratoire à partir des signaux d'objet générés.

2. Le procédé selon la revendication 1, comprenant en outre, après avoir retiré des objets de la bordure de chaque cadre d'image, définir une taille d'objet minimale proportionnelle à chaque taille de cadre d'image capturé ;
effacer les objets de chaque cadre d'image capturé inférieurs à la taille minimale d'objet définie.

3. Le procédé selon la revendication 1, dans lequel l'identification d'un ou de plusieurs objets comprend l'identification de points ou de lignes sur la au moins une partie d'une portion de la poitrine d'un conducteur.

4. Le procédé selon la revendication 1, dans lequel l'identification d'un ou de plusieurs objets sur chaque cadre d'image capturé comprend identifier des objets prédéterminés et mettre en correspondance ces objets avec des objets de référence.

5. Le procédé selon l'une quelconque des revendications 1 ou 3 à 4, dans lequel l'extraction d'une ou de plusieurs caractéristiques desdits un ou plusieurs objets comprend l'extraction d'un centroïde de chacun de l'un ou des plusieurs objets, respectivement.

6. Le procédé selon l'une quelconque des revendications 1 ou 3 à 5, dans lequel l'identification d'un ou de plusieurs objets comprend l'identification d'un ensemble de motifs uniques.

7. Le procédé selon la revendication 6, dans lequel l'identification d'un motif unique comprend l'extraction et la mise en correspondance de caractéristiques à partir d'une image de référence et des cadres d'image capturés.

8. Le procédé selon l'une quelconque des revendications 6 à 7, comprenant en outre le suivi des caractéristiques extraites et mises en correspondance au moyen d'un algorithme de flux optique.

9. Le procédé selon la revendication 8, dans lequel le suivi des caractéristiques extraites et mises en correspondance comprend le calcul du module pour chaque caractéristique suivie et mise en correspondance.

10. Un dispositif (10) pour extraire un signal respiratoire, comprenant :
Un moyen de capture de cadre d'image (2) pour capturer une série de cadres d'image d'au moins une portion de la poitrine d'un conducteur, dans lequel le moyen de capture de cadre d'image comprend un capteur d'image (15) ;
un moyen d'identification d'objets pour identifier un ou plusieurs objets sur chaque cadre d'image capturé, où l'identification d'un ou de plusieurs objets comprend :
effectuer une binarisation de chaque cadre d'image capturé avec un seuil ;
appliquer un filtre passe-haut à chaque cadre d'image capturé afin d'extraire des bordures du cadre d'image correspondant ;
effectuer une dilatation de chaque cadre d'image capturé de sorte que les bordures du cadre d'image correspondant soient compactées ;
retirer des objets des bordures de chaque cadre d'image capturé ;
annuler la dilatation de chaque cadre d'image capturé en lissant le cadre d'image correspondant avec un même élément structurel ;
appliquer une ouverture à chaque cadre d'image capturé ; un moyen d'extraction de caractéristiques pour extraire une ou plusieurs caractéristiques desdits un ou plusieurs objets pour chaque cadre d'image, respectivement ;
Un moyen de traitement d'image (3), le moyen de traitement d'image (3) comprenant :
un moyen de génération de signal d'objet, pour générer un signal d'objet à partir de la série de cadres d'image, dans lequel le moyen de génération de signal d'objet est configuré pour générer le signal d'objet à partir de la ou des caractéristiques extraites respectives pour chaque objet et à partir de la série de cadres d'image.
un moyen de construction de signal respiratoire, pour construire le signal respiratoire à partir des signaux d'objet générés.

11. Le dispositif selon la revendication 10, comprenant en outre :
un moyen pour définir une taille minimale d'objet proportionnelle à chaque taille de cadre d'image capturé, et
un moyen pour effacer les objets de chaque cadre d'image capturé inférieur à la taille minimale d'objet.

12. Le dispositif selon la revendication 10, dans lequel le moyen de traitement d'image comprend un moteur de traitement d'image en temps réel.

13. Le dispositif selon la revendication 10, dans lequel le moyen d'extraction d'objet comprend un élément calculateur de centroïde.

14. Le dispositif selon l'une quelconque des revendications 10 ou 13, dans lequel le moyen de génération de signal d'objet comprend un élément calculateur de distance pour identifier des paires de caractéristiques appartenant au même objet dans des cadres d'image capturées successivement.

15. Le dispositif selon l'une quelconque des revendications 10 ou 13 à 14, dans lequel le moyen de génération de signal comprend un filtre passe-bas pour recevoir le signal d'objet pour chaque objet et générer le signal respiratoire.
